# EUROPEAN PATENT SPECIFICATION

(11) **EP 3 240 583 B1**
(45) Date of publication and mention of the grant of the patent: **08.05.2019**
(21) Application number: 16708744.4
(22) Date of filing: 04.01.2016
(51) Int. Cl.: A61L 27/44, A61L 27/46, A61L 27/20, A61L 27/50, A61L 27/14

(54) **CROSS-LINKABLE TISSUE BULKING COMPOSITONS**
VERNETZBARE GEWEBEVERSTÄRKUNGSZUSAMMENSETZUNGEN
COMPOSITIONS DE COMBLEMENT TISSULAIRE RÉTICULABLES

(30) Priority: 02.01.2015 US 201562099371 P
(43) Date of publication of application: 08.11.2017
(73) Proprietor: Ankrum, James, Iowa City, IA 55125 (US); Cezo, James, D., Falcon Heights, MN 55113 (US); Kangas, Steven L., Woodbury, MN 55125 (US)
(72) Inventor: Ankrum, James, Iowa City, IA 55125 (US); Cezo, James, D., Falcon Heights, MN 55113 (US); Kangas, Steven L., Woodbury, MN 55125 (US)
(74) Representative: Peterreins Schley
(86) International application number: PCT/US2016/012032
(87) International publication number: WO 2016/109847

(56) References cited:
- EP-A2- 2 181 722
- WO-A1-2011/101684
- WO-A2-2013/037965
- US-A1- 2007 184 087
- DATABASE WPI Week 201501 Thomson Scientific, London, GB; AN 2014-W75732 XP002757867, & CN 104 069 485 A (HYBIO PHARM CO LTD) 1 October 2014 (2014-10-01)

## Description

### BACKGROUND

Tissue bulking compositions can be utilized in support and augmentation of a variety of tissues in various fields including cardiology, orthopedics, urology, gastroenterology, and cosmetic and reconstructive surgery, among others.

The present disclosure pertains to compositions, kits and methods that are useful in tissue bulking.

WO 2011/101684 describes a composition comprising pH-responsive cross-linked microgel particles, for use in repairing damaged or degenerated tissues. The microgel particles reach the intended site in collapsed configuration and there swell as a consequence of a change in pH and further cross-link to each other to form a hydrogel composed of the particles bound together.

### SUMMARY

In some aspects, the present disclosure pertains to tissue bulking compositions that comprise: (a) solid particles comprising a polymer that has a pH-dependent solubility such that the solid particles dissolve upon an increase or decrease in pH (also referred to herein as a "pH-sensitive polymer"), (b) a crosslinking agent for the pH-sensitive polymer and (c) a pH modifying agent that generates acid or base in vivo. Where the tissue bulking composition comprises solid particles that dissolve upon a decrease in pH, the pH modifying agent generates acid in vivo; where the tissue bulking composition comprises solid particles that dissolve upon an increase in pH, the pH modifying agent generates base in vivo. In certain embodiments, the solid particles may be less than 100 microns (µm) in size. In certain embodiments, which may be used in combination with any of the above aspects and embodiments, the tissue bulking compositions may be in the form of a dry powder (e.g. in a container that comprises a needle-penetrable septum, etc.), or the tissue bulking compositions may be in the form of an injectable dispersion that further comprises an aqueous or non-aqueous liquid.

In certain embodiments, which may be used in combination with any of the above aspects and embodiments, the tissue bulking compositions comprise solid particles comprising a pH-sensitive polymer that has a pH-dependent solubility such that the solid particles dissolve upon a decrease in pH, and the pH modifying agent that is selected is one that acts to generate acid in vivo. In certain alternative embodiments, which may be used in combination with any of the above aspects and embodiments, the tissue bulking compositions comprise solid particles comprising a pH-sensitive polymer that has a pH-dependent solubility such that the solid particles dissolve upon an increase in pH, and the pH modifying agent that is selected is one that generates base in vivo.

In certain embodiments, which may be used in combination with any of the above aspects and embodiments, the tissue bulking compositions further comprise inorganic filler particles.

In some aspects, the present disclosure pertains to kits that comprise (a) a tissue bulking composition as described in any of the above aspects and embodiments and (b) an injection device configured to inject the injectable dispersion into a mammalian subject. The injection device may be, for example, a needle injection catheter, among other possibilities.

In some aspects, the present disclosure pertains to methods of forming injectable dispersions. The methods comprise admixing a liquid vehicle with a tissue bulking composition as described in any of the above aspects and embodiments, thereby forming an injectable dispersion. In certain beneficial embodiments, the tissue bulking agent that is admixed with the liquid vehicle is in the form of a dry powder.

In some aspects, the present disclosure pertains to methods that comprise injecting an injectable dispersion into a subject, the injectable dispersion comprising a tissue bulking composition as described in any of the above aspects and embodiments. Such methods are useful, for example, in the augmentation of a variety of tissues in various fields including cardiology, orthopedics, urology, gastroenterology, and cosmetic and reconstructive surgery, among others.

In some embodiments the methods further comprise admixing a tissue bulking composition as described in any of the above aspects and embodiments with a liquid vehicle, thereby forming the injectable dispersion, prior to injecting the injectable dispersion into the subject.

In some embodiments, which may be used in combination with any of the above aspects and embodiments, the injectable dispersion may be delivered via a catheter.

In some embodiments, which may be used in combination with any of the above aspects and embodiments, the methods comprise injecting the injectable dispersion into a ventricular wall of a heart (e.g., in the treatment of heart failure). As discussed in more detail below, the present disclosure is advantageous, for example, in that bulking compositions may be provided, which have low viscosity upon injection and which can then build viscosity after injection by undergoing a polymer dissolution process in-situ, thereby resulting in formation of a viscous polymer gel solution. The crosslinking agent formulated into the composition can crosslink the polymer upon dissolution, leading to a stable hydrogel matrix.

The above and other aspects, embodiments and advantages of the present disclosure will become apparent to those of ordinary skill in the art upon review of the detailed description set forth below.

### BRIEF DESCRIPTION OF THE DRAWINGS

FIG. 1 is a schematic illustration showing modulus and viscosity (arbitrary units) for a hypothetical polymer solution;
FIGS. 2A and 2B are schematic illustrations showing a method of treatment, in accordance with an embodiment of the present disclosure;
FIG. 3 is a graph of viscosity versus percent solids for chitosan solutions (two lots) and a chitosan dispersion, in accordance with embodiments of the present disclosure;
FIG. 4 is a bar graph showing compression modulus for crosslinked chitosan hydrogels formed from two different lots of chitosan, in accordance with embodiments of the present disclosure;
FIG. 5 is a bar graph showing compression modulus for crosslinked calcium alginate, a genipin-crosslinked chitosan solution, and several genipin-crosslinked chitosan dispersions having varying amounts of chitosan, in accordance with embodiments of the present disclosure;
FIG. 6 is a bar graph showing compression modulus for crosslinked calcium alginate and several silica-containing genipin-crosslinked chitosan dispersions containing chitosan and glucono-delta-lactone in varying amounts, in accordance with embodiments of the present disclosure;
FIG. 7 is a bar graph showing compression modulus for crosslinked calcium alginate and several genipin-crosslinked chitosan dispersions containing silica in varying amounts for two cure times, in accordance with embodiments of the present disclosure;
FIG. 8 is a bar graph showing compression modulus for crosslinked calcium alginate and several genipin-crosslinked chitosan solutions containing hydroxyapatite in varying amounts, in accordance with embodiments of the present disclosure; and
FIG. 9 is a graph of viscosity versus shear rate for a 2 wt% chitosan solution and for a solution containing 2 wt% chitosan and 20 wt% hydroxyapatite, in accordance with embodiments of the present disclosure.

### DETAILED DESCRIPTION

A more complete understanding of the present disclosure is available by reference to the following detailed description of numerous aspects and embodiments of the disclosure. The detailed description which follows is intended to illustrate but not limit the disclosure.

As noted above, the present disclosure pertains to compositions, kits and methods that are useful in tissue bulking.

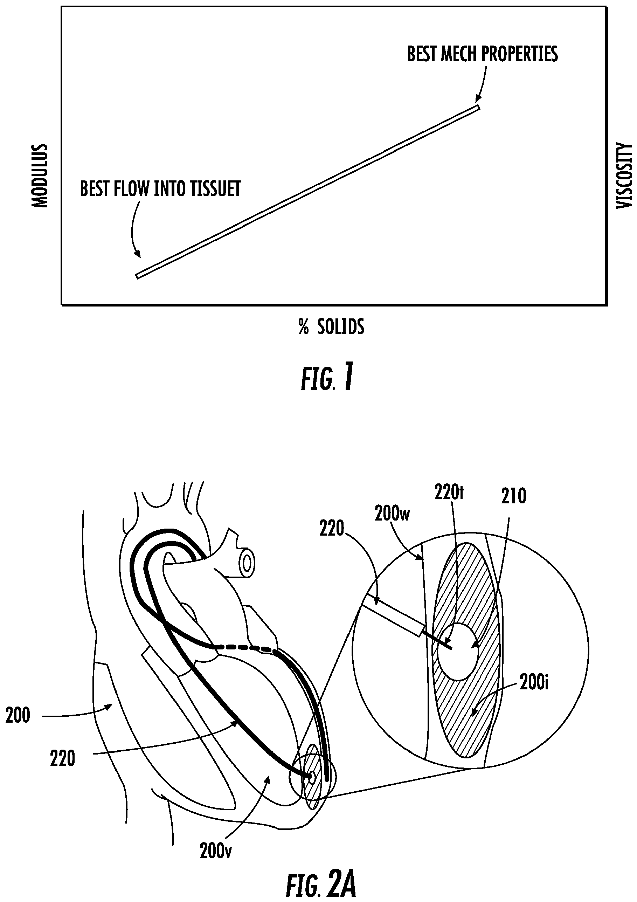

Current bulking compositions typically contain high molecular weight water soluble polymers and large amounts of water (e.g., 95-98 wt%). As a consequence, the hydrogel implants that are formed from the bulking compositions commonly have poor mechanical properties. Increasing the polymer content (% solids), while resulting in improved mechanical properties also dramatically increases viscosity, ultimately to the point where the bulking composition cannot be injected. The changes in modulus and viscosity (arbitrary units) with % solids for a hypothetical high molecular weight water soluble polymer is shown schematically in Fig. 1.

Thus, an ideal bulking composition would have high solids for high strength and low viscosity for ease of delivery and optimal penetration into interstices of the injected tissue. However, this is not possible with traditional polymer solutions for the reasons noted above.

The present disclosure describes bulking compositions that have low viscosity upon injection and which then build viscosity after injection by undergoing a polymer dissolution process in-situ, which results in the formation of a viscous polymer gel solution. A crosslinking agent formulated into the composition can crosslink the polymer upon dissolution, leading to a high strength hydrogel matrix. In some embodiments, a crosslinking agent is selected which, in addition to crosslinking the polymer, can crosslink collagen of the surrounding tissue to itself and to the injected polymer, thereby creating a crosslinked polymer/collagen matrix.

More particularly, in the present disclosure, a bulking composition in the form of an injectable dispersion is injected into a living subject. The injectable dispersion comprises (a) solid particles comprising a pH-sensitive polymer having pH-dependent solubility such that the solid particles dissolve upon an increase or decrease in pH, (b) a pH modifying agent that generates acid or base in vivo upon injection and (c) a crosslinking agent for the pH-sensitive polymer. In embodiments where the composition comprises solid particles that dissolve upon a decrease in pH, a pH modifying agent is selected that generates acid over time in-vivo. Conversely, in embodiments where pH-sensitive solid particles are selected which dissolve upon an increase in pH, a pH modifying agent is selected that generates base over time in-vivo.

One test for determining whether or not solid particles dissolve upon an increase or decrease in pH is to vary the pH of a 2 wt% suspension of the particles in water at 37°C using a suitable pH adjusting agent, such as NaOH to increase pH or HCl to decrease pH. Because suspensions are typically cloudy and solutions are typically clear, dissolution can be visually observed as a decrease in cloudiness (clarification) in most cases. Whether or not particle dissolution has occurred may also be determined using microscopy or a laser particle analyzer such as those sold by Malvern Instruments Ltd. Particle dissolution will also typically be associated with a significant change in viscosity (i.e., a viscosity increase of at least 2 times, frequently, at least 10 times, or more).

Examples of pH-sensitive polymers that have pH-sensitive solubility such that the solid particles formed using such polymers will dissolve upon an increase in pH are alginic acid polymers, carboxymethyl cellulose, cellulose acetate succinate, cellulose acetate phthalate, hydroxy propyl methyl cellulose phthalate, hydroxy propyl methyl cellulose acetate succinate, polyvinyl acetate phthalate, methyl methacrylate-methacrylic acid copolymers, shellac and cellulose acetate trimellitate. Examples of basic compounds capable of increasing pH in-situ are primary alkyl amines, quaternary ammonium compounds such as benzyltrimethyl ammonium hydroxide, sodium bicarbonate and sodium carbonate. These basic compounds can be incorporated into a microsphere, for example a polymer microsphere capable of swelling/dissolving after injection thereby releasing the basic compound.

Examples of pH-sensitive polymers that have pH-sensitive solubility such that the solid particles formed using such polymers will dissolve upon a decrease in pH include polymers that comprises amine groups (e.g., primary amine groups), such as chitosan, poly(N,N dialkylaminoethyl methacrylates), allylamine copolymers, polyethyleneimine and poly L-Lysine.

In certain beneficial embodiments, the pH-sensitive polymer is a polysaccharide that comprises primary amine groups, for example, a polysaccharide that comprises glucosamine units. A specific example of such a polymer is a copolymer comprising glucosamine and N-acetyl-glucosamine, more particularly, chitosan. Chitosan is a modified polysaccharide containing randomly distributed β-(1-4)-linked D-glucosamine and N-acetyl-D-glucosamine monomer units. Chitosan is produced commercially by the alkaline N-deacetylation of chitin, which is a cellulose-like polymer consisting primarily of unbranched chains of modified glucose, specifically N-acetyl-D-glucosamine. Chitosan is insoluble in water at neutral or basic pH. It is soluble at acidic pH. For example, chitosan is soluble in dilute acetic acid.

In certain embodiments, the pH-sensitive polymer selected will have a viscosity average molecular weight (Mᵥ) of at least 50,000, for example, ranging from 50,000 to 250,000, among other possibilities.

In certain embodiments, solid particles formed from one or more pH-sensitive polymers may comprise 50 wt% or more of one or more pH-sensitive polymers, for example, 50 wt% or more, 60 wt% or more, 70 wt% or more, 80 wt% or more, 90 wt% or more, 95 wt% or more, 97.5 wt% or more, even as high as 99 wt% or more of one or more pH-sensitive polymers.

In certain embodiments, the solid particles formed from the one or more pH-sensitive polymers may be less than 1000 microns in size, less than 250 microns in size, less than 100 microns in size, or even less than 25 microns in size. For example, at least 95% by weight of the solid particles in the compositions described herein may be less than 1000 microns in size, preferably at least 95% by weight of the particles may be less than 250 microns in size, and more preferably at least 99% by weight of the particles may be less than 100 microns in size. In certain embodiments, at least 90% by weight of the solid particles in the compositions described herein may be between 10 nm and 100 microns in size.

As used herein, the particle "size" refers to the longest dimension of a particle (e.g., the diameter of a sphere, the length of a filamentous particle, etc.). Particle size may be determined using methods known in the art, for example, microscopy or a laser particle analyzer such as those sold by Malvern Instruments Ltd.

Examples of pH modifying agents that generate acid over time in-vivo include esters, for instance, polyesters and cyclic esters, which hydrolyze in the presence of water to form hydroxyacids or a combination of multifunctional acids and multifunctional alcohols (e.g., diacids and diols). Particular examples of cyclic esters include cyclic esters formed from one or more hydroxyacid units. A specific example is a cyclic ester of gluconic acid, for instance, glucono-delta-lactone (GDL), 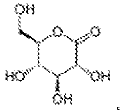 GDL is neutral, but hydrolyzes in water to form gluconic acid which is mild acid and therefore capable of dissolving pH-sensitive polymers such as chitosan.

Examples of crosslinking agents include crosslinking agents that are reactive with carboxyl, hydroxyl, amine and/or thiol groups within the pH-sensitive polymer that is selected (and within the tissue into which the composition is injected). In certain embodiments, a crosslinking agent is selected which is reactive with amine groups, for example, primary amine groups, that are present within suitable pH-sensitive polymers and tissue. Specific examples of such crosslinking agents include glutaraldehyde, formaldehyde, genipin, imidoesters and succinimides such as N-hydroxysuccinimide esters.

In certain beneficial embodiments, the crosslinking agent may be genipin, 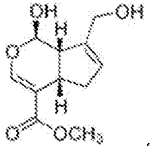 Genipin is a natural botanical product, which provides crosslinking of primary amine groups such as those found in collagen and fibrin at physiological pH and temperature. Genipin provides a comparable strength to aldehyde cross-linking without the toxicity associated with aldehydes. In fact, beneficial antioxidant and anti-inflammatory properties have been reported for genipin.

In certain embodiments, the compositions of the present disclosure may further comprise filler particles, for example, for purposes of increasing the modulus of the resulting crosslinked composition. Filler particles may be organic or inorganic. Examples of suitable filler particles include oxide ceramics (e.g., silicon dioxide, nanoclays and metal oxides such as iron oxide, titanium dioxide, alumina and zirconia), non-oxide ceramics including, calcium phosphate-based ceramics including hydroxyapatite, metals, metal alloys, and bioactive glass, and carbon nanotubes, among other possibilities.

In certain embodiments, the filler particles are less than 100 microns in size, less than 25 microns in size, than 10 microns in size, less than 2.5 microns in size, less than 1 micron in size, less than 0.25 micron in size, or even less than 0.1 micron in size. For example, 95% by weight of the filler particles in the compositions described herein may be less than 10 microns in size, preferably 95% by weight of the filler particles may be less than 2.5 microns in size, and more preferably 95% by weight of the filler particles may be less than 1 micron in size. In certain embodiments, at least 95% by weight of the solid particles in the compositions described herein may be between 1 nm and 1 micron in size.

In certain embodiments, the tissue bulking composition comprises solid particles comprising (a) a pH-dependent polymer, such as a polysaccharide, that comprises amine groups and that has a pH-dependent solubility in water such that the particles dissolve in water at 37°C upon a decrease in pH, (b) a crosslinking agent, such as glutaraldehyde, formaldehyde, genipin, imidoesters or succinimides, and (c) a pH modifying agent, such as a polyester or a cyclic ester, that generates acid in vivo.

In certain embodiments, the tissue bulking composition comprises solid particles comprising (a) a polysaccharide that comprises amine groups and that has a pH-dependent solubility in water such that the particles dissolve in water at 37°C upon a decrease in pH, (b) a crosslinking agent, such as glutaraldehyde, formaldehyde, genipin, imidoesters or succinimides, and (c) a polyester or a cyclic ester that generates acid in vivo.

In certain embodiments, the tissue bulking composition comprises solid particles comprising (a) a pH-dependent polymer, such as a polysaccharide, that comprises amine groups and that has a pH-dependent solubility in water such that the particles dissolve in water at 37°C upon a decrease in pH, (b) a crosslinking agent, such as glutaraldehyde, formaldehyde, genipin, imidoesters or succinimides, (c) a pH modifying agent, such as a polyester or a cyclic ester, that generates acid in vivo, and (d) inorganic or organic filler particles.

In certain embodiments, the compositions of the present disclosure may further comprise supplemental agents, for example, therapeutic agents and/or imaging contrast agents such as water based iodinated contrast agents or oil based contrast agents such as lipiodol, among other possibilities.

In some embodiments, the bulking compositions of the present disclosure are provided in the form of a dry powder that comprises (a) solid particles comprising a pH-sensitive polymer, (b) a pH modifying agent, (c) a crosslinking agent for the pH-sensitive polymer and (d) optionally, filler particles and/or other supplemental agents. The powder can be admixed with a suitable liquid vehicle to form an injectable dispersion immediately prior to injection. Examples of suitable liquids include aqueous liquids (e.g., water, saline, phosphate buffered saline, etc.) and non-aqueous liquids (e.g., glycerol, low molecular weight polyethylene glycols and oils such as mineral oil, vegetable oil, fatty acids, etc.). The powder can be provided in a sterile container, for example, in a container, such as a vial, that comprises a needle-penetrable septum.

In some embodiments, the bulking compositions of the present disclosure are provided in the form of an injectable dispersion that comprises (a) solid particles comprising a pH-sensitive polymer, (b) a pH modifying agent, (c) a crosslinking agent for the pH-sensitive polymer, (d) optionally, filler particles and/or other supplemental agents, and (e) a suitable aqueous or non-aqueous liquid. Where the pH modifying agent produces and acid or base upon exposure to water, the liquid is beneficially a non-aqueous liquid

In some embodiments, the bulking compositions of the present disclosure are provided in the form of (a) a first composition that comprises solid particles comprising a pH-sensitive polymer, optionally, filler particles and/or other supplemental agents, and optionally, a suitable liquid, and (b) a second composition comprising a pH modifying agent, a crosslinking agent for the pH-sensitive polymer, and optionally, a suitable liquid.

In the event that a pH modifying agent is selected which reacts with water (e.g., undergoes hydrolysis) in vivo to generate acid or base, it is beneficial in some embodiments to form a non-aqueous injection formulation. In other embodiments, an aqueous injection formulation may be formed in which the reaction kinetics of the reaction with water (e.g., hydrolysis) are sufficiently slow to permit injection to occur prior to the time where the viscosity of the formulation is significantly increased. In some embodiments, the pH sensitive solid particles are provided with a coating that is adapted to dissolve over a relatively short time frame (e.g., 5-30 minutes, etc.) after which the pH sensitive polymer of the solid particles can undergo dissolution and crosslinking. In addition to delaying the dissolution and crosslinking of the pH sensitive polymer, the coating material may also provide an initial increase in the viscosity of the composition upon delivery, thereby reducing washout.

In some embodiments, the bulking compositions of the present disclosure are provided in a kit which may comprise (a) solid particles comprising a pH-sensitive polymer, a pH modifying agent, a crosslinking agent for the pH-sensitive polymer, and optionally, filler particles and/or other supplemental agents, in one or more containers and (b) one or more of the following: (i) one or more containers of a suitable liquid (e.g., where the solid particles, pH modifying agent, crosslinking agent and/or optional agents are provided in dry form), for instance, one or more containers of an aqueous fluid such as water, saline or phosphate buffered saline or a non-aqueous fluid such as glycerol or an oil, and (ii) an injection device configured to inject the injectable dispersion to a subject (e.g., an injection catheter, hypodermic syringe and needle, etc.).

The methods, compositions and kits of the present disclosure are useful in a variety of tissue bulking procedures in a variety of fields. For example, the tissue bulking compositions may be used in the field of cardiology as a cardiac bulking agent, in the field of orthopedics for repair of cartilage defects, in the field of gastroenterology, wherein bulking of tissue at the gastro-esophageal junction can be used to treat gastro-esophageal reflux disease, to bulking of rectal tissue can be used to treat incontinence, or wherein the thickness of the stomach wall can be increased by tissue bulking, thereby decreasing the volume of the stomach to treat morbid obesity, in the field of urology, where placing bulking agent around the urethra at the neck of the urinary bladder can be used to ameliorate incontinence, and in cosmetic and reconstructive surgery wherein the tissue bulking compositions may be used for augmentation in the dermis or subdermis to treat skin contour deficiencies caused by various conditions and diseases, for beauty enhancement, for breast augmentation, or for treatment of regions of the body that need volume enlargement during reconstructive plastic surgery, such as after trauma or tumor resection.

Thus, while the tissue bulking compositions of the present disclosure are described in detail herein for use as cardiac bulking agents, it is to be understood that the compositions are not limited to such use, but rather are useful in a variety of medical fields.

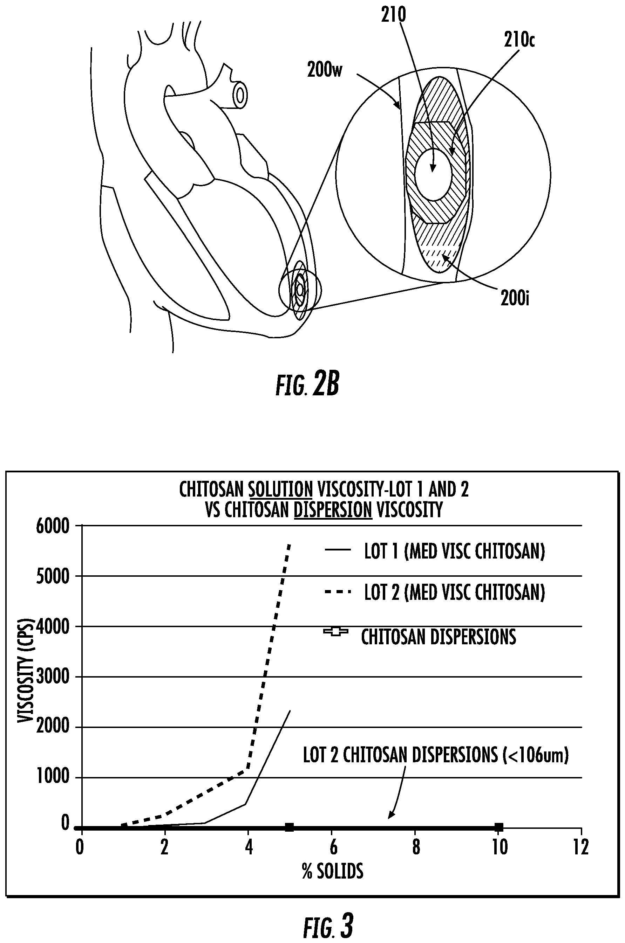

With regard to cardiac bulking, the tissue bulking compositions may be placed in the ventricular wall of the heart to thicken and strengthen the ventricle wall to prevent ventricular dilation and treat heart failure. While both transepicardial and transendocardial deployments of tissue bulking composition are feasible, minimally invasive transendocardial delivery via catheter deployment is preferred in some embodiments. Turning now to Fig. 2A, a human heart 200 is shown in which a catheter (e.g., a myocardial injection catheter, such as Stilletto™, Boston Scientific Inc., Boston, MA, USA) has been advanced to the left ventricular chamber 200v. A tip 220t of the injection catheter 220 is inserted into infarcted or near infarcted tissue 200i within the ventricular wall 200w where a volume of tissue bulking composition 210 is injected. After injection, the pH modifying agent reacts in vivo for modify the pH of the environment surrounding the pH-sensitive solid particles, causing the pH-sensitive polymer (and thus the pH-sensitive solid particles) to dissolve, thus forming a highly viscous mass. Dissolution of the pH-sensitive polymer makes the pH-sensitive polymer available for crosslinking by the crosslinking agent. In certain embodiments, a portion of the crosslinking agent diffuses from the volume of tissue bulking composition 210 into surrounding tissue, where the crosslinking agent acts to crosslink a portion 210c of the infarcted or near-infracted tissue 200i as shown in Fig. 2B. If desired, multiple injections may be performed to create multiple volumes of the tissue bulking composition 210 in the ventricular wall 200w.

For these and other applications, the material acts as an injectable hydrogel precursor that has properties and kinetics sufficient to remain in a relatively low viscosity fluid form within the catheter during delivery, while allowing the formation of a crosslinked hydrogel within the tissue following delivery. This allows the composition to be of sufficiently low viscosity to allow for successful injection, after which the composition increases in viscosity and crosslinks so as to more effectively retain the composition at the at the delivery site.

In certain embodiments, upon crosslinking, the compositions described herein form hydrogels having a compression modulus ranging from 25 kPa to 2500 kPa or more, for example, ranging from 25 kPa to 50 kPa to 100 kPa to 250 kPa to 500 kPa to 1000 kPa to 2500 kPa (i.e., ranging between any two of the preceding numerical values), for instance, beneficially ranging from 250 kPa to 2500 kPa in some embodiments.

Upon injection into an infarct zone of the heart, the bulking composition will increase the wall thickness and the crosslinking agent will crosslink the bulking agent to itself. Moreover, in some embodiments, the crosslinking agent may crosslink the bulking agent to the infarcted tissue as well as to crosslink the infarcted tissue to itself. By crosslinking damaged tissue, the native scaffolding may be reinforced. In this regard, the crosslinking effect is believed to improve the mechanical strength of the infarcted tissue, which in turn is believed to reduce dilation associated with post-myocardial-infarction remodeling, thereby offsetting dilated cardiomyopathy. Thus the compositions described herein may cause increased infarct wall thickness due to bulking agent per se as well as an increase in mechanical reinforcement of myocardium due the presence of the high modulus hydrogel and an increase in tensile strength of myocardium due to tissue crosslinking in some embodiments, which will resist elongation (dilation).

In certain specific embodiments, injectable compositions of the present disclosure are provided which comprise chitosan as a pH sensitive polymer. As noted above, chitosan is insoluble in water at neutral or basic pH, and it is soluble at acidic pH. Chitosan particles may be combined with a suitable crosslinking agent such as genipin (e.g., in a suitable container such as a vial). Genipin is capable of crosslinking polymers that have primary amine functionality such as chitosan and collagen. A latent acid generator, for example glucono-delta- lactone (GDL) may also be included. GDL is a cyclic ester and when dissolved in water hydrolyzes to gluconic acid. Various detailed Examples based on these three components (chitosan, genipin and GDL) are set forth below. Just prior to injection, an aqueous fluid such as water, saline or PBS may be added to a container containing the three components. The genipin and GDL dissolve, and the chitosan, being insoluble at neutral pH is dispersed in particulate form (i.e., it exists in the form of a dispersion). The dispersed chitosan particles add little to the solution viscosity. The dispersion is then injected into the tissue. Without being bound by theory, it is believed that within a few minutes the GDL begins to hydrolyze to gluconic acid, thereby reducing the pH and causing the chitosan to swell and dissolve, which increases the viscosity. As the chitosan dissolves, the genipin begins to crosslink the chitosan resulting in a crosslinked hydrogel. In addition, the genipin is also capable of crosslinking surrounding collagen to itself and to the chitosan, thereby forming a chitosan-collagen composite.

### Example 1. Preparation and evaluation of high strength chitosan hydrogel

2.0 g chitosan powder (low viscosity grade, Aldrich Chemical), 0.80 g GDL (Aldrich Chemical) and 0.029 g genipin (Wako Chemicals USA) were combined in a vial. 7.2 g of deionized (DI) water was added and the vial was shaken to dissolve the genipin and GDL and to disperse the chitosan powder. The low viscosity slurry was added to a petri dish and sealed. The petri dish was placed in an incubator at 37°C for 18-24 hrs. The dispersion visibly begins to gel within about 1 hr. The formulation of other gels with varying chitosan loadings are shown in Table 1.

**Table 1. Chitosan dispersion formulations**

| **Chitosan dispersions** | | | | | |
|---|---|---|---|---|---|
| | **Chitosan powder** | **DI water** | **Glucono delta lactone** | **Genipin** | **Total** |
| | **g** | **g** | **g** | **g** | **g** |
| | | | | | |
| **2.5% Chitosan** | **0.25** | **9.5** | **0.20** | **0.037** | **10.0** |
| **5% Chitosan** | **0.50** | **9.1** | **0.40** | **0.037** | **10.0** |
| **10% Chitosan** | **1.00** | **8.6** | **0.40** | **0.035** | **10.0** |
| **15% Chitosan** | **1.50** | **7.9** | **0.60** | **0.033** | **10.0** |
| **18% Chitosan** | **1.85** | **7.4** | **0.74** | **0.037** | **10.0** |

It has been found that there is considerable variability in viscosity from lot to lot for a given grade of chitosan. For example the viscosity specifications for low viscosity grade from Aldrich is 20-300cps at 1% solids in 1% acetic acid. Two different lots of the low viscosity grade of chitosan were evaluated, designated as lot 1 and lot 2. The two lots differ significantly in viscosity upon dissolution in acetic acid with lot 2 being significantly higher. FIG. 3 shows the solution viscosity (dissolved in acetic acid) of the two lots. The viscosity of the chitosan dispersions (<106 µm particle diameter) is shown for comparison. This shows that the dispersions possess very low viscosity compared to solutions of dissolved chitosan. In this regard, at 5% solids, the solution viscosity is 3700X greater for the lot 2 solution relative to the dispersion. Solutions at 10% solids could not be prepared due to exceedingly high viscosity.

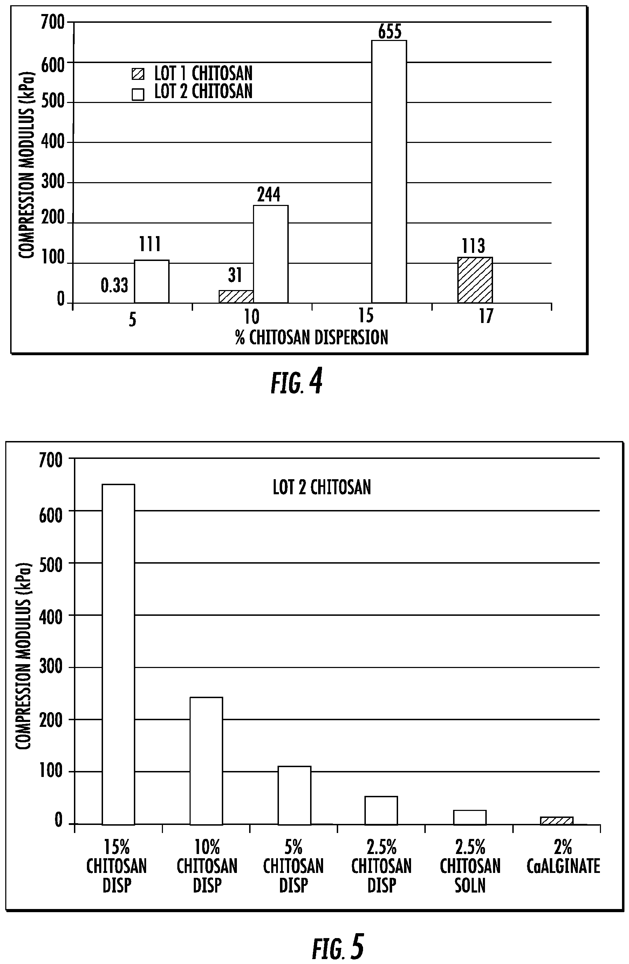

Compression modulus was testing on the cured gels using an MTS Systems Corporation tensile tester configured with a flat circular stainless steel punch tip with a surface area of 0.044mm². The probe tip was pressed into the gel at a speed of 6 mm/min to a depth of 1mm. The compression modulus was calculated from the slope of the resulting force/displacement plot. FIG. 4 shows the compression modulus of lot 1 and 2 chitosan crosslinked gels. The higher viscosity chitosan (which is believed to be a result of higher molecular weight) leads to gels with significantly greater compression modulus compared to the lower viscosity chitosan. FIG. 5 shows the compression modulus of chitosan dispersions (lot 2) prepared with varying chitosan content. Increasing the % chitosan solids in the dispersion results in significant increase in compression modulus. The compression modulus was measured on a calcium alginate (CaAlginate) gel as a comparative control. The CaAlginate control was prepared by first adding a 2% solids aqueous solution of sodium alginate (NaAlginate) to a petri dish. The surface of the alginate solution in the petri dish was sprayed with a fine mist of 2% CaCl₂ in water to crosslink the surface of the alginate solution. This allows one to then dispense a solution of 2% CaCl₂ over the surface of the alginate without disrupting the surface of the alginate. The alginate in the petri dish was incubated overnight at 37C and the excess CaCl₂ solution was decanted off the crosslinked alginate gel. The 15% chitosan based dispersion has a compression modulus that is 45X greater than the CaAlginate comparative control.

### Example 2. Preparation and evaluation of high strength chitosan silica nanocomposites

0.40 g GDL and 0.0261 g genipin were dissolved in 5.70g of aqueous Ludox silica nanodispersion (30 wt% silica) (LUDOX® AM colloidal silica and LUDOX® AS colloidal silica) (Sigma Aldrich) and 2.88g DI water. 1.0g chitosan powder was then added to the silica dispersion and shaken. The resulting low viscosity dispersion was poured into a petri dish, sealed and placed in an incubator at 37°C overnight or for 96 hr. Compression modulus was measured on the resulting crosslinked gels.

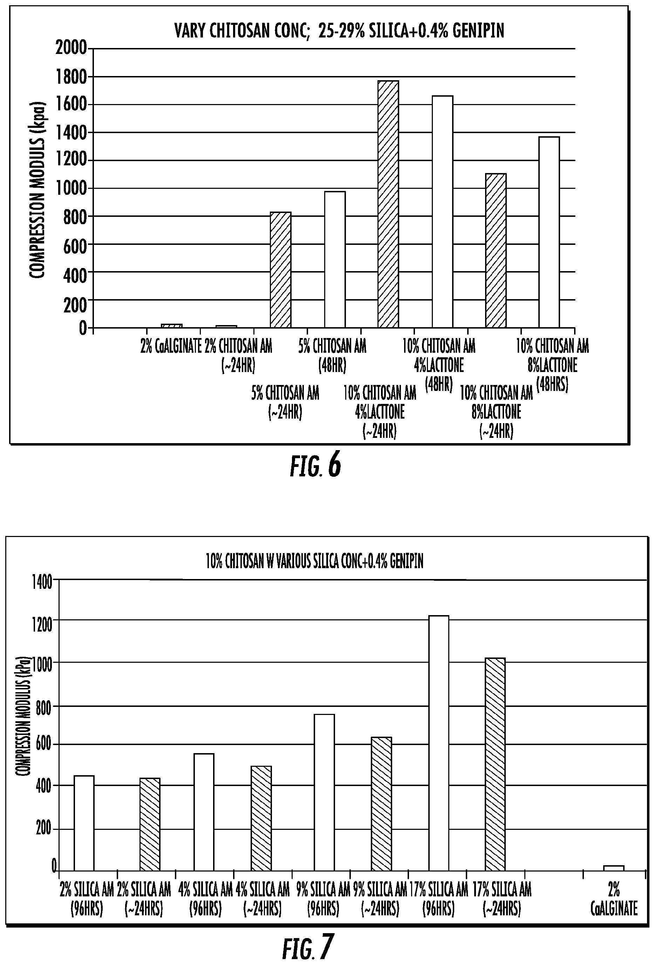

The preceding sample comprises 10 wt% chitosan, 17 wt% silica and 4% GDL (10-17-4). Other samples were also formed with the following wt% amounts of chitosan, silica and GDL, respectively: 2-29-2, 5-27-4, 10-26-4 and 10-25-8. FIG. 6 shows the compression modulus results for CaAlginate and the last four of these samples (where the silica was held between 25-29%, the chitosan dispersion loading was 2, 5 or 10 wt%, the GDL was 4 or 8 wt%, and the cure time was 24 or 48 hours). There was a slight increase in modulus in extending the cure from 24 hr to 48 hr, showing that the gels primarily cure within 24 hours at 37°C. Gels prepared with 4 w% GDL gave higher modulus than those prepared with 8 wt% GDL. This was somewhat surprising in that the stoichiometric quantity of GDL required to solubilize the chitosan was calculated to be about 8%. Increasing chitosan loading resulted in an increase in modulus. It is noted that the 10% chitosan sample was found to have a compression modulus 118x greater than the calcium alginate comparative control.

Another sample set comprised formulations in which the chitosan loading was held constant at 10wt% and the silica loading was varied from about 2-17 wt%. (Specifically, samples were formed with the following wt% amounts of chitosan, silica and GDL, respectively: 10-2-4, 10-4-4, 10-9-4, 10-17-4). The compression modulus for this sample set is shown in FIG. 7. There is slight increase in modulus in curing for 96 hours vs 24 hours. Moreover, modulus increased with increasing silica loading. Silica loadings of 26% gave the highest modulus (see FIG. 6), however there is tradeoff between modulus and dispersion viscosity in that a paste was formed at the 26% level which was easily deformable, but did not flow. The 2-17% dispersions initially possessed low viscosity and flowed easily. The 17% silica sample has a compression modulus 67x greater than the calcium alginate comparative control.

### Example 3. Preparation and evaluation of high strength chitosan hydroxyapatite nanocomposites

This Example differs from Examples 1 and 2 in that one starts with a low solids, low viscosity chitosan solution. The gel strength is generated primarily through addition of hydroxyapatite nanoparticles.

In one exemplary procedure, 0.040 g genipin was dissolved along with 0.2 g chitosan in 7.8g of 2wt% acetic acid. 2.0g HA was added to the chitosan solution and the dispersion was sonicated for ∼1 min to disperse the HA. The resulting low viscosity dispersion was poured into a petri dish, sealed and incubated at 37°C for overnight. This sample contained 2 wt% chitosan, 4 wt% genipin and 20 wt% HA.

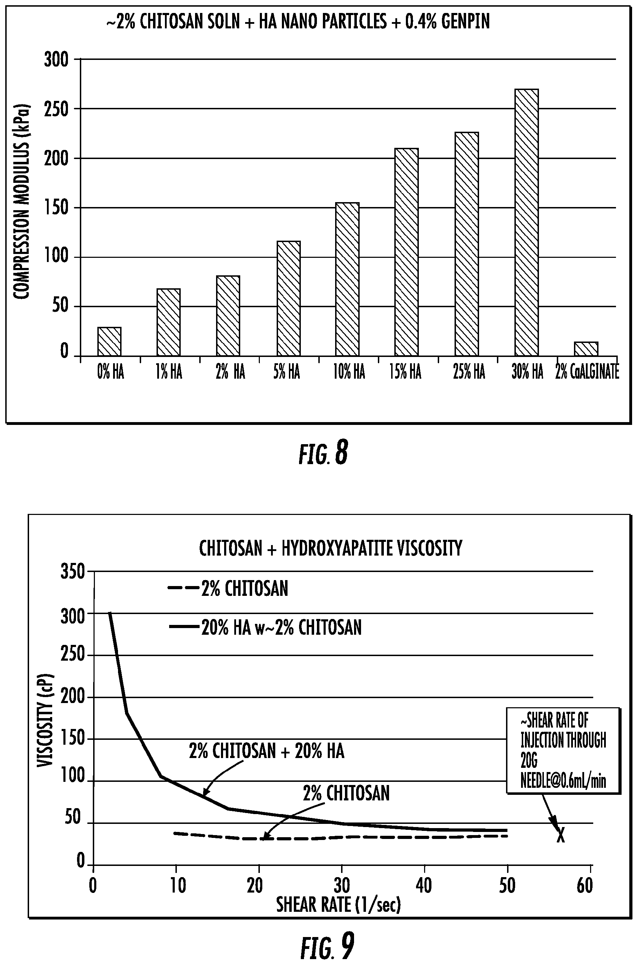

Additional samples were formed in an analogous fashion containing approximately 2wt% chitosan (1.8-2.5 wt%) in 2% acetic acid, approximately 4 wt% genipin (3.4-4.0 wt%), and 1, 2, 5, 10, 15, 25 or 30 wt% HA. Compression modulus was measured for the crosslinked gels. FIG.8 shows the compression modulus results of the formulations prepared. Compression modulus, which increases with increasing HA solids, can thus be tailored by varying the HA loading. Modulus values up to 18x higher than the calcium alginate control are possible.

### Example 4. Evaluation of chitosan and chitosan/hydroxyapatite dispersions

A chitosan solution was formed which contained 0.2 g chitosan dissolved in 2wt% acetic acid (2 wt% chitosan). A chitosan/HA dispersion was formed in which 2.0g HA was added to 0.2 g chitosan dissolved in 2wt% acetic acid (2 wt% chitosan, 20 wt% HA), and the dispersion was sonicated for ∼1 min to disperse the HA.

Viscosity as a function of shear rate was measured for the solution and dispersion using a Brookfield cone and plate viscometer, and the results are shown in FIG. 9. The chitosan solution with no HA shows Newtonian viscosity, i.e., no change in viscosity with shear rate. The chitosan dispersion containing 20% HA shows non-Newtonian behavior in the form of shear thinning. At very low shear rates the viscosity increases - this is beneficial to slow particle settling in solution. When the solution is shaken or injected through a catheter the viscosity approaches that of the chitosan solution with no HA. Shear thinning behavior is also beneficial in that it improves dispersion retention after sample injection.

## Claims

1. A tissue bulking composition comprising: (a) solid particles comprising a pH-sensitive polymer that has a pH-dependent solubility such that the solid particles dissolve upon an increase or decrease in pH, (b) a crosslinking agent for the pH-sensitive polymer and (c) a pH modifying agent that generates acid or base in vivo, wherein when the tissue bulking composition comprises solid particles that dissolve upon a decrease in pH, the pH modifying agent generates acid in vivo and wherein when the tissue bulking composition comprises solid particles that dissolve upon an increase in pH, the pH modifying agent generates base in vivo.

2. The tissue bulking composition of claim 1, wherein the solid particles are less than 100 microns (µm) in size.

3. The tissue bulking composition of any one of claims 1-2, wherein the tissue bulking composition is in the form of a dry powder or wherein the tissue bulking composition is an injectable dispersion that further comprises an aqueous or non-aqueous liquid.

4. The tissue bulking composition of any one of claims 1-3, wherein the solid particles comprise a pH-sensitive polymer that dissolves upon a decrease in pH.

5. The tissue bulking composition of claim 4, wherein the pH-sensitive polymer comprises amine groups.

6. The tissue bulking composition of claim 4, wherein the pH-sensitive polymer is a polysaccharide that comprises amine groups.

7. The tissue bulking composition of any one of claims 4-6, wherein the pH modifying agent is a polyester or a cyclic ester.

8. The tissue bulking composition of any one of claims 4-7, wherein the crosslinking agent is an agent that crosslinks amine groups.

9. The tissue bulking composition of claim 8, wherein the crosslinking agent is genipin.

10. The tissue bulking composition of any one of claims 1-3, wherein the solid particles comprise a pH-sensitive polymer that dissolves upon an increase in pH.

11. The tissue bulking composition of any one of claims 1-10, further comprising inorganic filler particles.

12. A kit comprising: a tissue bulking composition in accordance with any one of claims 1-11 and an injection device.

13. The kit of claim 12, wherein the injection device is a needle injection catheter.

14. The kit of claim 12, wherein the tissue bulking composition is in the form of a dry powder that is disposed in a container having a needle-penetrable septum.

15. A method of forming an injectable dispersion comprising admixing (a) a tissue bulking composition in accordance with any one of claims 1-11 with (b) an aqueous or non-aqueous liquid vehicle, thereby forming said injectable dispersion.

## Patentansprüche

1. Gewebeverstärkungszusammensetzung, umfassend: (a) Feststoffteilchen, die ein pH-empfindliches Polymer, das eine pH-abhängige Löslichkeit aufweist, umfassen, so dass sich die Feststoffteilchen bei einer Erhöhung oder Abnahme des pH-Werts auflösen, (b) ein Vernetzungsmittel für das pH-empfindliche Polymer und (c) ein pH-Modifizierungsmittel, das in vivo eine Säure oder Base erzeugt, und wobei, wenn die Gewebeverstärkungszusammensetzung Feststoffteichen umfasst, die sich bei einer Abnahme des pH-Werts auflösen, das pH-Modifizierungsmittel in vivo eine Säure erzeugt, und wobei wenn die Gewebezusammensetzung Feststoffteilchen umfasst, die sich bei einer Erhöhung des pH-Werts auflösen, das pH-Modifizierungsmittel in vivo eine Base erzeugt.

2. Gewebeverstärkungszusammensetzung nach Anspruch 1, wobei die Feststoffteilchen eine Größe von weniger als 100 Mikron (µm) aufweisen.

3. Gewebeverstärkungszusammensetzung nach einem der Ansprüche 1 bis 2, wobei die Gewebeverstärkungszusammensetzung in Form eines trockenen Pulvers vorliegt oder wobei die Gewebeverstärkungszusammensetzung einer injizierbare Dispersion ist, die zudem eine wässrige oder nicht-wässrige Flüssigkeit umfasst.

4. Gewebeverstärkungszusammensetzung nach einem der Ansprüche 1 bis 3, wobei die Feststoffteilchen ein pH-empfindliches Polymer umfassen, das sich bei einer Abnahme des pH-Werts auflöst.

5. Gewebeverstärkungszusammensetzung nach Anspruch 4, wobei das pH-empfindliche Polymer Amingruppen umfasst.

6. Gewebeverstärkungszusammensetzung nach Anspruch 4, wobei das pH-empfindliche Polymer ein Polysaccharid ist, das Amingruppen umfasst.

7. Gewebeverstärkungszusammensetzung nach einem der Ansprüche 4 bis 6, wobei das den pH-Wert modifizierende Mittel ein Polyester oder ein cyclischer Ester ist.

8. Gewebeverstärkungszusammensetzung nach einem der Ansprüche 4 bis 7, wobei das Vernetzungsmittel ein Mittel ist, das Amingruppen vernetzt.

9. Gewebeverstärkungszusammensetzung nach Anspruch 8, wobei das Vernetzungsmittel Genipin ist.

10. Gewebeverstärkungszusammensetzung nach einem der Ansprüche 1 bis 3, wobei die Feststoffteilchen ein pH-empfindliches Polymer umfassen, das sich bei einer Erhöhung des pH-Werts auflöst.

11. Gewebeverstärkungszusammensetzung nach einem der Ansprüche 1 bis 10, zudem umfassend anorganische Füllstoffteilchen.

12. Kit, umfassend: eine Gewebeverstärkungszusammensetzung nach einem der Ansprüche 1 bis 11 und eine Injektionsvorrichtung.

13. Kit nach Anspruch 12, wobei die Injektionsvorrichtung ein Nadelinjektionskatheter ist.

14. Kit nach Anspruch 12, wobei die Gewebeverstärkungszusammensetzung in Form eines trockenen Pulvers vorliegt, das in einem Behälter angeordnet ist, der ein nadelgängiges Septum aufweist.

15. Verfahren zum Bilden einer injizierbaren Dispersion, umfassend das Vermischen von (a) einer Gewebeverstärkungszusammensetzung nach einem der Ansprüche 1 bis 11 mit (b) einem wässrigen oder nicht-wässrigen flüssigen Träger, wodurch die injizierbare Dispersion gebildet wird.

## Revendications

1. Composition de gonflement tissulaire composée : (a) de particules solides comprenant un polymère sensible au pH, dont la solubilité dépend du pH de sorte que les particules solides se dissolvent lors d'une hausse ou d'une baisse du pH, (b) d'un agent réticulant le polymère sensible au pH et (c) d'un agent de modification du pH qui produit de l'acide ou une base *in vivo*, où, lorsque la composition de gonflement tissulaire comprend des particules solides se dissolvant lors d'une baisse du pH, l'agent de modification du pH produit de l'acide *in vivo* et, lorsque la composition de gonflement tissulaire comprend des particules solides se dissolvant lors d'une hausse du pH, l'agent de modification du pH produit une base *in vivo*.

2. Composition de gonflement tissulaire selon la revendication 1, les particules solides étant d'une grosseur inférieure à 100 microns (µm).

3. Composition de gonflement tissulaire selon l'une quelconque des revendications 1-2, la composition de gonflement tissulaire se trouvant sous forme de poudre sèche ou la composition de gonflement tissulaire étant une dispersion injectable comprenant en outre un liquide aqueux ou non aqueux.

4. Composition de gonflement tissulaire selon l'une quelconque des revendications 1-3, les particules solides comprenant un polymère sensible au pH se dissolvant lors d'une baisse du pH.

5. Composition de gonflement tissulaire selon la revendication 4, le polymère sensible au pH comprenant des groupes amine.

6. Composition de gonflement tissulaire selon la revendication 4, le polymère sensible au pH étant un polysaccharide comprenant des groupes amine.

7. Composition de gonflement tissulaire selon l'une quelconque des revendications 4-6, l'agent de modification du pH étant un polyester ou un ester cyclique.

8. Composition de gonflement tissulaire selon l'une quelconque des revendications 4-7, l'agent de réticulation étant un agent qui réticule les groupes amine.

9. Composition de gonflement tissulaire selon la revendication 8, l'agent de réticulation étant la génipine.

10. Composition de gonflement tissulaire selon l'une quelconque des revendications 1-3, les particules solides comprenant un polymère sensible au pH se dissolvant lors d'une hausse du pH.

11. Composition de gonflement tissulaire selon l'une quelconque des revendications 1-10 comprenant en outre des particules de charge inorganiques.

12. Trousse comprenant : une composition de gonflement tissulaire selon l'une quelconque des revendications 1-11 et un dispositif d'injection.

13. Trousse selon la revendication 12, le dispositif d'injection étant un cathéter d'injection à aiguille.

14. Trousse selon la revendication 12, la composition de gonflement tissulaire étant sous forme de poudre sèche se trouvant dans un récipient équipé d'un septum dans lequel une aiguille peut pénétrer.

15. Procédé de préparation d'une dispersion injectable comprenant le mélange (a) d'une composition de gonflement tissulaire selon l'une quelconque des revendications 1-11 avec (b) un véhicule liquide aqueux ou non aqueux, formant ainsi ladite dispersion injectable.
